# EUROPEAN PATENT SPECIFICATION

(11) **EP 3 963 315 B1**
(45) Date of publication and mention of the grant of the patent: **07.06.2023**
(21) Application number: 20722384.3
(22) Date of filing: 28.04.2020
(51) Int. Cl.: G01N 25/04, G01N 33/28, B01L 7/02

(54) **DEVICE FOR DETERMINING PROPERTIES OF HYDROCARBON FLUIDS AND SIMILAR SUBSTANCES**
VORRICHTUNG ZUR BESTIMMUNG VON EIGENSCHAFTEN VON KOHLENWASSERSTOFFFLÜSSIGKEITEN UND ÄHNLICHEN SUBSTANZEN
DISPOSITIF DE DÉTERMINATION DES PROPRIÉTÉS DE FLUIDES HYDROCARBONÉS ET DE SUBSTANCES SIMILAIRES

(30) Priority: 02.05.2019 IT 201900006482
(43) Date of publication of application: 09.03.2022
(73) Proprietor: Chimec S.p.A., 00144 Roma (IT)
(72) Inventor: MANTARRO, Milena, 00128 Roma (IT); CIARDI, Umberto, 03025 Monte San Giovanni Campano (FR) (IT); BRUNO, Massimo, 00071 Pomezia (RM) (IT); VOLPONI, Andrea, 00132 Roma (IT); ARCA, Simone, 05100 Terni (IT); D'ALESSANDRO, Emilio, 65012 Cepagatti (PE) (IT)
(74) Representative: Romano, Giuseppe
(86) International application number: PCT/IB2020/053993
(87) International publication number: WO 2020/222119

(56) References cited:
- CN-A- 105 854 972
- CN-A- 109 482 251
- US-A- 3 201 970
- US-A- 4 770 540

## Description

The present invention relates to a device for determining properties of hydrocarbon fluids and other similar substances.

The need for determining the physical and chemical properties of the hydrocarbon fluids and, generally, for determining the physical and chemical properties of similar substances such as lubricants and other oily substances is known.

In particular, the need for determining the pour point of the hydrocarbon fluids, that is the temperature below which the substance is no more able to pour down, is known. The determination of the pour point takes place by cooling down a sample of substance to be examined with the purpose of searching for the corresponding temperature value at which the substance solidifies and does not pour down anymore.

In particular, for the hydrocarbons, after a preliminary heating, the sample is cooled down with a defined cooling speed and the pouring at intervals of 3°C is controlled. The temperature is monitored by means of a bulb thermometer immersed just below the surface of the sample under examination.

The lowest temperature at which the motion of the sample surface is observed, before the complete locking, corresponds to the "pour point" value.

There are specific regulations defining the standards to be met during the tests for determining the pour point, for example ASTM D97 regulation and ASTM D5853 regulation.

The above-mentioned tests are performed by means of suitable devices.

A first type of known devices provides thermostatic baths in which at least a sample of substance to be examined is to be inserted.

The thermostatic baths are of the type of ethylene glycol or alcohol baths and they are set at temperatures ranging from 27°C to -33°C, in particular values equal to 27°C, 0°C, -18°C, -33°C.

The determination of the pour point can take place both manually and automatically.

The manual determination is based upon the observation of the sample by an operator. On the contrary, the automatic determination takes place by means of instruments, for example optical instruments, installed in the device and able to detect the motion of the substance to be examined with the purpose of recognizing when this is no more able to pour down.

This first type of devices has drawbacks.

A first drawback is linked to the fact that high amounts of ethylene glycol or alcohol for the operation of the apparatuses, from a minimum of 5 litres up to 10 litres, are required.

The high amounts of ethylene glycol and alcohol affect the operation costs, by increasing them, and limit the use of such devices.

Another drawback of this first type of devices is that they result to be cumbersome and, then, they cannot be transported from a place to another one.

Consequently, the samples to be analysed have to be moved from the extraction/collection site, to the site in which the device is placed, with the risk that their chemical-physical features can vary.

In fact, it is known that the hydrocarbon fluids are subjected to a so-called "ageing" process due to the moving away of the light fractions, of the oxidation of the hydrocarbon fractions and other phenomena linked to the chemical-physical nature of the sampled substance.

The sample aging involves an alteration in the chemical and physical properties which makes the determination of the pour point less reliable.

A second type of known devices provides a transportable container in which a thermostatic bath with refrigerating fluid, for example ethylene glycol, alcohol, water or other refrigerating fluids, is placed. The cooling system, for this second type of known devices, can be of Peltier type or compressors.

In the thermostatic bath a sample of substance to be examined can be inserted for determining the pour point.

A drawback of this second type of known devices is linked to the fact that they can perform one single test at a time.

For this reason, the above-described second type of devices results to be a little suitable when the sample analyses require a lot of time or when there is the need for analysing several samples at different temperatures.

Moreover, in the sites for collecting the samples the time available for the analyses is limited and this makes the use of such second type of devices unfavourable. CN105854972A and CN109482251A describe a heating equipment based on a thermostatic water bath with only heating function.

US4770540A describes a process for determining the turbidity point of a liquid.

The main task of the present invention is to develop a device for determining properties of hydrocarbon fluids and similar substances capable of performing several measurements at the same time.

An object of the present invention is to develop a device for determining properties of hydrocarbon fluids and similar substances which could ease the procedures for determining the pour point of a hydrocarbon fluid or similar substance.

Another object of the present invention is to develop a device for determining properties of hydrocarbon fluids and similar substances which has to be cheap and easy to be used. An additional object of the present invention is to have a machine which can be transported by plane since its weight is less than 32kg.

The above-illustrated objects are reached by the present inventive device having the features of claim 1.

Other features and advantages of the present invention will result better evident from the description of a preferred, but not exclusive, embodiment of a device for determining properties of hydrocarbon fluids and similar substances, illustrated by way of example, but not with limitative purposes, in the enclosed figures of drawings wherein:
figure 1 is an axonometric view of the device according to the invention with lid in opening position;
figure 2 is an axonometric view of the device according to the invention with lid in closing position;
figure 3 is an axonometric view of the device according to the invention with containing casing in transparency.

With particular reference to such figures, 1 designates globally a device for determining properties of hydrocarbon fluids and similar substances.

The device 1 comprises a containing casing 2 provided with a containment base 3 and with a lid 4 associated to the containment base 3 for closing it.

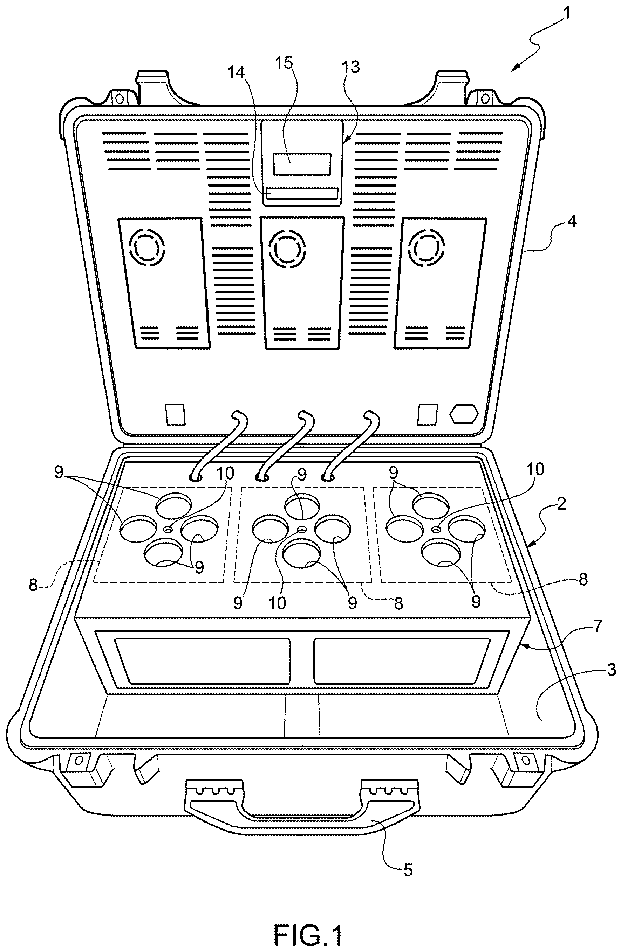

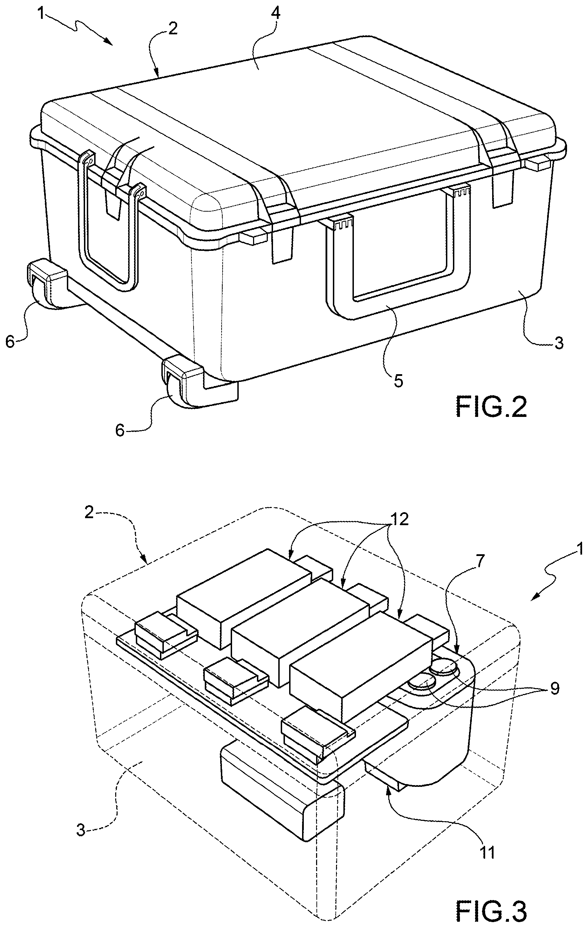

In particular, the lid 4 can move from an opening position (figure 1), wherein the containment base 3 is accessible, and a closing position (figure 2), wherein the lid 4 prevents the access to the containment base 3.

In the present embodiment, the containing casing 2 has a gripping handle 5 and it is shaped like a suitcase, preferably of trolley type, thus equipped with an extractable (retractable) handle on the side opposite to the wheels.

Usefully, the containing casing 2 can include wheels 6 apt to allow the sliding thereof along at least a predefined direction.

Such features allow to transport easily the containing casing 2.

Advantageously, the containing casing 2 comprises a covering structure comprising technopolymer layers.

In this way, the containing casing 2 results to be thermally insulated, apart from guaranteeing a protection of the internal components from hits.

The device 1 comprises thermoregulating means 11, 12 inserted in the containing casing 2.

The thermoregulating means 11, 12 is described in details hereinafter in the present description.

Moreover, the device 1 comprises a working unit 7 in which samples containing hydrocarbon fluids and similar substances to be analysed can be inserted.

The working unit 7 is housed in the containment base 3 and it is associated to the thermoregulating means 11, 12 to induce a variation in temperature of the samples. According to the invention, the working unit 7 comprises a plurality of thermostatic baths 8 each one having at least a housing seat 9 for one of the above-mentioned samples.

Still according to the invention, the thermostatic baths 8 are associated with the thermoregulating means 11, 12 independently from one another to induce different temperature variations on each one of the samples housed in the thermostatic baths 8. In this way, it is possible to perform analyses on different samples by bringing them to different temperatures, independently from one another.

Then, the advantage of being able to perform several tests at the same time is obtained, so as to shorten, for example, the periods of time required to determine the pour point of the examined hydrocarbon fluid.

In the present description the determination of the pour point of hydrocarbon fluids is mainly referred to, but the same considerations can relate to the determination of other properties of hydrocarbon fluids, for example the cloud point or other properties variable when the substance temperature varies.

In the illustrated embodiment, the working unit 7 comprises three thermostatic baths 8 independent from one another and made of aluminium.

Different embodiments are not excluded wherein, for example, the working unit 7 is made of another light material having high thermal conductivity, such as a copper. Embodiments are not excluded too, wherein the working unit 7 comprises a number of thermostatic baths 8 different from three, for example two or four.

Usefully, the working unit 7 can comprise detection seats 10 in which detection elements, for example probes, thermometers or other similar devices, useful to detect the temperature of the same and thermostatic baths, can be inserted.

Advantageously, the thermostatic baths 8 comprise a plurality of housing seats 9 in which a respective plurality of samples to be subjected to the same temperature variation can be housed.

In particular, the thermostatic baths 8 illustrated in the figures comprise, each one, four housing seats 9.

In this way, it is possible housing four different samples in one same thermostatic bath 8 in order to subject them to the same temperature variation.

Preferably, the shape and the sizes of the housing seats 9 comply with what provided by ASTM D97 and ASTM D5853 technical regulations, so as to guarantee the same cooling kinetics of the sample under examination. In this way a total exactness of the results obtained with the invention and with the International reference regulations is guaranteed.

Advantageously, the thermoregulating means 11, 12 comprises a plurality of Peltier elements 11 each one associated to a respective thermostatic bath 8 for its cooling/heating *(for sake of simplicity Peltier elements placed in contact with the bottom of the thermal bath were illustrated).*

In particular, the thermoregulating means 11, 12 provides a Peltier element 11 placed in contact with each thermostatic bath 8 to guarantee a uniform temperature in the same thermostatic bath.

In the present embodiment, the thermoregulating means 11, 12 can include three Peltier elements 11 placed in contact with the respective thermostatic baths 8.

The Peltier elements 11 which can be found in the figures are placed in contact with the bottom of the thermal baths 8, but solutions are not excluded, in which the Peltier elements 11 are placed in contact with the side walls, or are placed in another point of the thermostatic bath 8.

Alternative solutions are not excluded, for example in which the thermoregulating means 11, 12 comprises several Peltier elements 11 associated with each thermostatic bath 8.

Advantageously, the thermoregulating means 11, 12 comprises cooling means, for sake of simplicity not illustrated in the figures, associated with the Peltier elements 11 and wherein a cooling fluid, for example glycol, or alcohol, or water, or a mixture thereof, apt to remove heat from the same Peltier elements, is operative.

By applying a current to the Peltier elements 11, they absorb/release heat from/to the thermostatic bath 8 and release/absorb heat to/from the heat exchange cooling means on liquids such as glycol, alcohol, water or a mixture thereof.

The Peltier elements allow to implement a compact and light thermal exchange system, by allowing the device 1 to fall within a weight less than 32 kg, suitable for the air transport.

Moreover, the use of the Peltier elements reduces the need for maintenance and the weakness of the device 1, and it improves the resistance and reliability thereof.

For these reasons, the use of the Peltier elements 11 is more advantageous, for example, than the use of a gas refrigerated machine, or another known cooling system. Usefully, the thermoregulating means 11, 12 comprises a plurality of electronic supports 12 housed in the lid 4 and operatively connected with respective Peltier elements 11 for the management and control of temperature variations to be induced in the thermostatic baths 8.

The function of the electronic supports 12 is that of managing the operation of the Peltier elements 11 with the purpose of adjusting its thermal power, keeping in temperature, variation in temperature, switching on and switching off.

Since they are housed in the lid 4, the electronic supports 12 are safeguarded against possible accidental errors of the operator, for example accidental pouring of chemical products on the supports themselves, and they are sheltered from the portions working in temperature, by preventing the contact thereof.

Advantageously, the device 1 comprises condensate collection means, for sake of simplicity not illustrated in the figures, located in the containment base 3.

In this way the device 1 can work under extreme environmental conditions, for example desert heat or high humidity.

In these cases, in fact, the operation of refrigerating systems involves a high risk of accumulating condensate.

The condensate collection means placed in the containment base 3 allows to adjust the outflow thereof, by preventing possible malfunctions linked to the contact between the condensate water and the Peltier elements 11 or other portions of the device 1.

The device 1 comprises electronic management and control means inserted into the lid 4.

Usefully, the electronic management and control means is operatively connected with the thermoregulating means 11, 12 and with the working unit 7.

In particular, the electronic management and control means comprises a data processing unit, for sake of simplicity not illustrated in the figures, operatively connected with the thermoregulating means 11, 12 and with the working unit 7 so as to receive and process data, thereamong data relating to the operation temperatures. Usefully, the data processing unit can be operatively connected with the electronic supports 12 so as to receive data on the operation of the Peltier elements 11, for example temperature values, thermal power, information about the switching-on or switching-off status of the Peltier element, etc.

Analogously, the data processing unit can be operatively connected with the electronic supports 12 so as to send to the same electronic supports 12 data for controlling the operation of the Peltier elements 11, for example temperature values to be obtained, thermal power, switching-on and switching-off input.

Usefully, the electronic management and control means comprises at least a user interface unit 13.

The user interface unit 13 is operatively associated to the data processing unit.

In this way, an operator, by interacting with the user interface unit 13, can enter information and data useful to the management and to the control of the electronic supports 12 and, then, of the operation of the Peltier elements 11, by setting the several parameters for performing the tests to be carried out on the samples.

Usefully, the user interface unit 13 can include an analogue keyboard 14 and a reading screen 15, but different configurations are not excluded, for example touchscreen screens or other integrated solutions.

Advantageously, the electronic management and control means can include at least an output unit which can be operatively connected with one or more external peripherals, for example pc, tablet, smartphone or other electronic devices.

The output unit is operatively connected with the data processing unit.

The device 1, then, can be managed via software by connecting the output unit with any compatible peripheral unit.

The output unit can be of the type of a USB port, or of an electromagnetic-wave transmitting-receiving element, such as a wi-fi antenna or a Bluetooth, or infrared antenna.

The electronic management and control means comprises a protection unit adapted to deactivate the device upon the occurrence of malfunction conditions.

In the present description, under the term "malfunction conditions" all conditions are meant which could generate malfunctions in the device, for example voltage overloads, overheating, lack of coolant, etc.

Usefully, the device 1 can comprise sensors able to detect the amount of coolant and thermal probes.

Both sensors and probes can be associated with the data processing unit, the latter being able to determine the occurrence or not of the malfunction conditions through a processing of the data coming from the sensors and probes.

The operation of the present invention is as follows.

An operator opens the lid 4 by making the working unit 7 placed in the containment base 3 accessible.

The samples to be analysed are inserted in the housing seats 9 and they are subjected to gradually decreasing temperatures until the temperature value corresponding to the pour point is determined.

The thermostatic baths 8 can be brought to predefined temperatures, independently from one another, by means of the respective Peltier elements 11.

Each Peltier element 11 absorbs heat from the thermostatic bath 8, by causing a progressive decrease in temperature of the thermostatic bath 8 and, then, of the sample to be analysed.

The Peltier elements 11, in turn, release heat to the coolant circulating in the cooling means associated thereto.

The operator can set the temperature values to be obtained through the user interface 13 or through software from external peripheral unit connected to the output unit.

In each case, by setting the temperature data it is possible to manage and control the temperature variations by means of the data processing unit operatively connected with the electronic supports 12.

The thermostatic baths 8 can be brought to different temperatures from one another, so as to perform different tests at the same time.

Wholly analogous considerations can be made for determining chemical-physical parameters depending from the temperature of the analysed sample.

Practically it has been noted that the described invention meets the proposed objects and in particular it is underlined that the device for determining properties of hydrocarbon fluids and similar substances is able to perform several analyses at the same time.

The working unit having a plurality of thermostatic baths, each one thereof able to be heated/cooled independently from one another, allows to perform several analyses at the same time on several samples.

In this way it is possible to decrease the time required for determining properties of the examined substance, for example of the pour point.

The developed device, moreover, eases the procedures for determining the pour point or other properties of a substance, the transportability and the use at extreme environmental conditions being eased.

The developed device results to be cheap and easy to be used.

The Peltier elements, in fact, can be easily found on the market and, with respect to known cooling systems, require less maintenance and lower volumes of coolants. Other advantages obtained by the developed device are the following:
- having available compact and transportable instruments, with weight less than the maximum allowed for a hold baggage (32kg);
- external casing resistant to hits and watertight for protecting the electronic components;
- use flexibility, the instruments being able to be used wherever there is a socket, thus even when the monitoring of ongoing chemical treatments is required;
- possibility of performing analyses on several samples at the same time and parallelly, with time and cost saving;
- independent thermostatic baths, with temperature programmable in the range from +50°C to - 33°C and possibility of performing analyses according to ASTM D97 and ASTM D5853 regulations;
- possibility of using tailored management software for controlling the temperatures of the single thermostatic baths, for checking and managing the power of the cooling/heating systems and for the delayed switching-on and switching-off programming.
- maximum insulation of instruments, in this way one limits the waste of energy which is then used to the maximum for cooling/heating the samples, apart from guaranteeing high operational safety for the user.

## Claims

1. A device (1) for determining properties of hydrocarbon fluids and similar substances comprising:
- a containing casing (2) provided with a containment base (3) and a lid (4) associated with said containment base (3) for closing it;
- thermoregulating means (11, 12) inserted in said containing casing (2);
- a working unit (7) wherein samples containing hydrocarbon fluids and similar substances to be analysed can be inserted, housed in said containment base (3) and associated to said thermoregulating means (11, 12) to induce a variation in temperature of said samples;
said working unit (7) comprising a plurality of thermostatic baths (8) each one having at least a housing seat (9) for one of said samples, said thermostatic baths (8) being associated with said thermoregulating means (11, 12) independently from one another to induce different temperature variations on each one of said samples,
the device being **characterized in that** it comprises electronic management and control means inserted in said lid (4).

2. The device (1) according to claim 1, **characterized in that** said thermostatic baths (8) comprise a plurality of said housing seats (9) wherein a respective plurality of said samples to be subjected to the same temperature variation can be housed.

3. The device (1) according to one or more of the previous claims, **characterized in that** said thermoregulating means (11, 12) comprises a plurality of Peltier elements (11) each one associated with a respective thermostatic bath (8) for its cooling/heating.

4. The device (1) according to one or more of the previous claims, **characterized in that** said thermoregulating means (11, 12) comprises a plurality of electronic supports (12) housed in said lid (4) and operatively connected with said respective Peltier elements (11) for the management and control of temperature variations.

5. The device (1) according to claim 3 or 4, **characterized in that** said thermoregulating means (11, 12) comprises cooling means associated with said Peltier elements (11) and wherein a cooling fluid is operative apt to remove heat from said Peltier elements.

6. The device (1) according to one or more of the previous claims, **characterized in that** it comprises condensate collection means placed in said containment base (3).

7. The device (1) according to one or more of the previous claims, **characterized in that** said containment casing (2) comprises a covering structure comprising technopolymer layers.

8. The device (1) according to one or more of the previous claims, **characterized in that** said electronic management and control means comprises at least a data processing unit operatively connected with said thermoregulating means (11, 12) and with said working unit (7).

9. The device (1) according to one or more of the previous claims, **characterized in that** said electronic management and control means comprises at least a user interface unit (13).

10. The device (1) according to one or more of the previous claims, **characterized in that** said electronic management and control means comprises at least an output unit which can be operatively connected with one or more external peripherals.

11. The device (1) according to one or more of the previous claims, **characterized in that** said electronic management and control means comprises a protection unit adapted to deactivate the device upon the occurrence of malfunction conditions.

12. The device (1) according to one or more of the previous claims, **characterized in that** it is shaped like a suitcase, preferably of trolley type.

13. The device (1) according to one or more of the previous claims, **characterized in that** it has a weight less than or equal to 32 Kg.

## Patentansprüche

1. Vorrichtung (1) zur Bestimmung von Eigenschaften von Kohlenwasserstoffflüssigkeiten und ähnlichen Substanzen, umfassend:
- ein Aufnahmegehäuse (2), das mit einem Aufnahmeunterteil (3) und einem Deckel (4) versehen ist, der dem Aufnahmeunterteil (3) zum Verschließen desselben assoziiert ist;
- Wärmeregulierungsmittel (11, 12), die in das Aufnahmegehäuse (2) eingesetzt sind;
- eine in dem Aufnahmegehäuse (3) untergebrachte und den Thermoregulierungsmitteln (11, 12) assoziierte Arbeitseinheit (7), in die Proben, die zu analysierende Kohlenwasserstoffflüssigkeiten und ähnliche Substanzen enthalten, eingesetzt werden können, um eine Veränderung der Temperatur der Proben zu bewirken;
wobei die Arbeitseinheit (7) eine Vielzahl von thermostatischen Bädern (8) umfasst, von denen jedes mindestens einen Gehäusesitz (9) für eine der Proben aufweist, wobei die thermostatischen Bäder (8) unabhängig voneinander mit den Thermoregulierungsmitteln (11, 12) assoziiert sind, um unterschiedliche Temperaturschwankungen bei jeder der Proben zu bewirken,
wobei die Vorrichtung **dadurch gekennzeichnet ist, dass** sie elektronische Verwaltungs- und Steuermittel umfasst, die in den Deckel (4) eingesetzt sind.

2. Vorrichtung (1) nach Anspruch 1, **dadurch gekennzeichnet, dass** die thermostatischen Bäder (8) eine Vielzahl von den Gehäusesitzen (9) umfassen, wobei eine jeweilige Vielzahl der Proben, die der gleichen Temperaturschwankung unterworfen werden sollen, aufgenommen werden kann.

3. Vorrichtung (1) nach einem oder mehreren der vorhergehenden Ansprüche, **dadurch gekennzeichnet, dass** die Wärmeregulierungsmittel (11, 12) eine Vielzahl von Peltier-Elementen (11) umfassen, von denen jedes mit einem jeweiligen thermostatischen Bad (8) zu dessen Kühlung/Erwärmung assoziiert ist.

4. Vorrichtung (1) nach einem oder mehreren der vorhergehenden Ansprüche, **dadurch gekennzeichnet, dass** die Wärmeregulierungsmittel (11, 12) eine Vielzahl von elektronischen Trägern (12) umfassen, die in dem Deckel (4) aufgenommen sind und mit den jeweiligen Peltier-Elementen (11) zur Verwaltung und Steuerung von Temperaturschwankungen in Wirkverbindung stehen.

5. Vorrichtung (1) nach Anspruch 3 oder 4, **dadurch gekennzeichnet, dass** die Wärmeregulierungsmittel (11, 12) Kühlmittel umfassen, die mit den Peltier-Elementen (11) assoziiert sind, und wobei ein Kühlmittel dazu ausgelegt ist, Wärme von den Peltier-Elementen zu entfernen.

6. Vorrichtung (1) nach einem oder mehreren der vorhergehenden Ansprüche, **dadurch gekennzeichnet, dass** sie Kondensatsammelmittel umfasst, die in dem Aufnahmeunterteil (3) angeordnet sind.

7. Vorrichtung (1) nach einem oder mehreren der vorhergehenden Ansprüche, **dadurch gekennzeichnet, dass** das Aufnahmegehäuse (2) eine Abdeckstruktur mit Technopolymerschichten umfasst.

8. Vorrichtung (1) nach einem oder mehreren der vorhergehenden Ansprüche, **dadurch gekennzeichnet, dass** die elektronischen Verwaltungs- und Steuermittel mindestens eine Datenverarbeitungseinheit umfassen, die mit den Wärmeregulierungsmitteln (11, 12) und mit der Arbeitseinheit (7) in Wirkverbindung stehen.

9. Vorrichtung (1) nach einem oder mehreren der vorhergehenden Ansprüche, **dadurch gekennzeichnet, dass** die elektronischen Verwaltungs- und Steuermittel mindestens eine Benutzerschnittstelleneinheit (13) umfassen.

10. Vorrichtung (1) nach einem oder mehreren der vorhergehenden Ansprüche, **dadurch gekennzeichnet, dass** die elektronischen Verwaltungs- und Steuermittel mindestens eine Ausgabeeinheit umfassen, die mit einem oder mehreren externen Peripheriegeräten in Wirkverbindung gebracht werden kann.

11. Vorrichtung (1) nach einem oder mehreren der vorhergehenden Ansprüche, **dadurch gekennzeichnet, dass** die elektronischen Verwaltungs- und Steuermittel eine Schutzeinheit umfassen, die dazu eingerichtet ist, die Vorrichtung beim Auftreten von Fehlfunktionsbedingungen zu deaktivieren.

12. Vorrichtung (1) nach einem oder mehreren der vorhergehenden Ansprüche, **dadurch gekennzeichnet, dass** sie wie ein Koffer, vorzugsweise vom Typ Trolley, geformt ist.

13. Vorrichtung (1) nach einem oder mehreren der vorhergehenden Ansprüche, **dadurch gekennzeichnet, dass** sie ein Gewicht von höchstens 32 kg aufweist.

## Revendications

1. Dispositif (1) pour déterminer des propriétés de fluides hydrocarbonés et de substances similaires comprenant :
- un boîtier de rétention (2) doté d'une base de rétention (3) et d'un couvercle (4) associé à ladite base de rétention (3) pour sa fermeture ;
- des moyens de thermorégulation (11, 12) insérés dans ledit boîtier de rétention (2) ;
- une unité de travail (7) dans laquelle des échantillons contenant des fluides hydrocarbonés et des substances similaires devant être analysés peuvent être insérés, logée dans ladite base de rétention (3) et associée audit moyen de thermorégulation (11, 12) pour induire une variation de température desdits échantillons ;
ladite unité de travail (7) comprenant une pluralité de bains thermostatiques (8) ayant chacun au moins un siège de logement (9) pour l'un desdits échantillons, lesdits bains thermostatiques (8) étant associés auxdits moyens de thermorégulation (11, 12) indépendamment les uns des autres pour induire différentes variations de température sur chacun desdits échantillons,
le dispositif étant **caractérisé en ce qu'**il comprend des moyens de commande et de gestion électroniques insérés dans ledit couvercle (4).

2. Dispositif (1) selon la revendication 1, **caractérisé en ce que** lesdits bains thermostatiques (8) comprennent une pluralité desdits sièges de logement (9), dans lequel une pluralité respective desdits échantillons devant être soumis à la même variation de température peut être logée.

3. Dispositif (1) selon une ou plusieurs des revendications précédentes, **caractérisé en ce que** lesdits moyens de thermorégulation (11, 12) comprennent une pluralité d'éléments Peltier (11) associés chacun à un bain thermostatique respectif (8) pour son refroidissement/ chauffage.

4. Dispositif (1) selon une ou plusieurs des revendications précédentes, **caractérisé en ce que** lesdits moyens de thermorégulation (11, 12) comprennent une pluralité de supports électroniques (12) logés dans ledit couvercle (4) et connectés de manière opérationnelle auxdits éléments Peltier respectifs (11) pour la gestion et la commande de variations de température.

5. Dispositif (1) selon la revendication 3 ou 4, **caractérisé en ce que** lesdits moyens de thermorégulation (11, 12) comprennent des moyens de refroidissement associés auxdits éléments Peltier (11), et dans lequel un fluide de refroidissement est apte de manière opérationnelle à éliminer la chaleur desdits éléments Peltier.

6. Dispositif (1) selon une ou plusieurs des revendications précédentes, **caractérisé en ce qu'**il comprend des moyens de collecte de condensat placés dans ladite base de rétention (3).

7. Dispositif (1) selon une ou plusieurs des revendications précédentes, **caractérisé en ce que** ledit boîtier de rétention (2) comprend une structure de recouvrement comprenant des couches en polymère technique.

8. Dispositif (1) selon une ou plusieurs des revendications précédentes, **caractérisé en ce que** lesdits moyens de gestion et de commande électroniques comprennent au moins une unité de traitement de données connectée de manière opérationnelle auxdits moyens de thermorégulation (11, 12) et à ladite unité de travail (7).

9. Dispositif (1) selon une ou plusieurs des revendications précédentes, **caractérisé en ce que** lesdits moyens de gestion et de commande électroniques comprennent au moins une unité d'interface utilisateur (13).

10. Dispositif (1) selon une ou plusieurs des revendications précédentes, **caractérisé en ce que** lesdits moyens de gestion et de commande électroniques comprennent au moins une unité de sortie qui peut être connectée de manière opérationnelle à un ou plusieurs périphériques externes.

11. Dispositif (1) selon une ou plusieurs des revendications précédentes, **caractérisé en ce que** lesdits moyens de gestion et de commande électroniques comprennent une unité de protection adaptée pour désactiver le dispositif lors de l'apparition de conditions de mauvais fonctionnement.

12. Dispositif (1) selon une ou plusieurs des revendications précédentes, **caractérisé en ce qu'**il a la forme d'une valise, de préférence de type à roulettes.

13. Dispositif (1) selon une ou plusieurs des revendications précédentes, **caractérisé en ce qu'**il a un poids inférieur ou égal à 32 kg.
